(19) Europäisches Patentamt
European Patent Office
Office européen des brevets

(11) **EP 4 480 424 A1**

(12) **EUROPEAN PATENT APPLICATION**

(43) Date of publication:
**25.12.2024 Bulletin 2024/52**

(21) Application number: **23180865.0**

(22) Date of filing: **22.06.2023**

(51) International Patent Classification (IPC):
**A61B 8/08** *(2006.01)*     **A61B 8/00** *(2006.01)*

(52) Cooperative Patent Classification (CPC):
**A61B 8/4254; A61B 8/085; A61B 8/4245;**
**A61B 8/4263; A61B 8/4416;** A61B 8/461;
A61B 8/463; A61B 8/54

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **V, Manikanda Krishnan**
**Eindhoven (NL)**
• **SETH, Subhendu**
**Eindhoven (NL)**
• **ANURADHA, Anuradha**
**5656AG Eindhoven (NL)**

(74) Representative: **Philips Intellectual Property &**
**Standards**
**High Tech Campus 52**
**5656 AG Eindhoven (NL)**

(54) **PROVIDING ULTRASOUND IMAGING GUIDANCE**

(57)     A mechanism for identifying a recommended path for an ultrasound probe during an ultrasound imaging process. An image of a surface region, covering a desired sub-surface region, is received (211). The image is processed to identify (212) the recommended path for the ultrasound probe that would capture ultrasound image data of the sub-surface region.

FIG. 2

EP 4 480 424 A1

**Description**

FIELD OF THE INVENTION

The present invention relates to the field of ultrasound imaging, and in particular to the provision of guidance for an ultrasound imaging process.

BACKGROUND OF THE INVENTION

Ultrasound imaging continues to be an important non-invasive imaging procedure for assessing the condition of a subject. Ultrasound imaging is often considered to be of particular importance in the field of obstetrics, to monitor fetal growth and/or condition. However, ultrasound imaging finds use in other areas as well, particularly cardiac or esophageal ultrasound imaging. One attractive quality of ultrasound imaging is its portability, as the equipment required to perform ultrasound imaging is relatively compact and requires less complex equipment and fewer power resources compared to other imaging modalities.

Indeed, ultrasound imaging has fast become an important diagnostic or assessment tool in low-resource regions in many parts of the world. One problem that these low-resource regions face is a lack of well-trained sonographers to acquire and interpret ultrasound scans.

There is therefore a strong desire to increase and improve the guidance available to a user or operator of an ultrasound imaging probe, to improve the capturing of ultrasound images of a desired sub-surface region. Capturing sufficient data is essential for accurate and reliable interpretation of a patient's condition.

Accordingly, there is a need for improved mechanisms for providing ultrasound imaging guidance.

SUMMARY OF THE INVENTION

The invention is defined by the independent claims. The dependent claims define advantageous embodiments.

In an aspect of the invention, there is provided a computer-implemented method for providing ultrasound imaging guidance. The computer-implemented method comprises performing an image display process comprising: receiving (e.g. from a camera) an image of a surface region along which an ultrasound probe is to be moved during an ultrasound imaging procedure; processing the image of the surface region to identify a path along the surface region for the ultrasound probe, wherein if moved along the identified path during the ultrasound imaging procedure, the ultrasound probe would capture a sequence of ultrasound images covering a desired sub-surface region; controlling a user interface to display the image; and augmenting the display of the image with a representation of the identified path that overlays the representation of the surface region in the displayed image.

Embodiments thereby provide a mechanism for defining or identifying a recommended path (the identified path) to be taken by an ultrasound probe by processing an image of the surface region over which the ultrasound probe is to be moved, is moving or has moved when performing an ultrasound imaging process. This provides a low-cost and adaptive technique for generating ultrasound guidance information that can be employed even in low-resource environments, e.g., in which processing power and/or guidance equipment is limited.

In some examples, the method comprises iteratively performing the image display process, wherein the image received in each iteration of the image display process comprises a different image in a sequence of images of the surface region. In this way, the recommended path can be produced for each frame of a video.

In some examples, the next image in a sequence of images comprises the most recently available image of a video stream of the surface region. This approach is particularly advantageous to provide ongoing guidance throughout the course of an ultrasound imaging procedure, e.g., to adapt the path to the most recently available image of the ultrasound imaging procedure to help guide the operator of the ultrasound probe.

The method may further comprise tracking a movement of the ultrasound probe during the ultrasound imaging procedure. The image display process may further comprise: processing the tracked movement of the ultrasound probe to identify an actual path along the surface region taken by the ultrasound probe; and augmenting the display of the image with a representation of the identified actual path that overlays the representation of the surface region in the display image.

This approach provides feedback for an operator of the ultrasound probe as to their success in following the (recommended) identified path. This information can, for instance, be used during the ultrasound imaging procedure to correct the path of the ultrasound probe or to repeat missed parts of the (recommended) identified path. Alternatively, this information can be used after completion of the ultrasound imaging process to teach or train an operator of the ultrasound probe, e.g., to assess the performance of the operator. In this way, for instance, a measure of utility or usefulness for any obtained ultrasound data can be defined.

The step of processing the tracked movement of the ultrasound probe may comprise using movement data captured by a movement sensor in the ultrasound probe to track the movement of the ultrasound probe.

In some examples, the method further comprises: iteratively receiving an ultrasound image captured by the ultrasound probe during the ultrasound imaging procedure; and for each ultrasound image: processing the ultrasound image to determine whether or not a region of interest is captured in the ultrasound image; and responsive to determining that a region of interest is captured, controlling the user interface to provide a user-perceptible alert that the ultrasound probe is capturing an ultrasound image of a region of interest.

Thus, in this approach, a check is made to determine if there are interesting anatomies (e.g., anatomical features, elements or structures) that have been detected in the particular frame of an ultrasound imaging process. If a positive determination is made, then an audio, visual and/or haptic feedback (e.g., a notification beep, alert box etc.) may be sent the operator. This may prompt the operator, for instance, to keep the probe in that region so as to acquire additional ultrasound image data about the anatomy of interest.

This approach thereby increases a likelihood that useful ultrasound data about a particular region of interest will be obtained. In particular, this approach advantageously alerts the operator when the probe is positioned over a region of interest. This, in itself, provides useful information to the operator (e.g., to make a clinical decision if the region of interest is in an unusual location or the like).

In some examples, the step of processing the image of the surface region comprises processing the image using an ellipsoid fitting algorithm to fit an ellipsoid over the portion of the image representing the surface region; and identifying the path based at least on a size of the fitted ellipsoid.

In some examples, the step of identifying the path based on at least a size of the fitted ellipsoid comprises using one or more known characteristics of the ultrasound probe.

In some examples, the one or more known characteristics of the ultrasound probe comprises a maximum distance.

The maximum distance may be a recommended (maximum) distance between sweep lines, e.g., as recommended by a manufacturer of the ultrasound probe.

The maximum distance may be a maximum allowable distance between a first position of the ultrasound probe, for imaging a first sub-surface region, and a second position of the ultrasound probe, for imaging a second sub-surface region, in which one side of the first sub-surface region wholly overlaps the second sub-surface region.

In some examples, the step of identifying the path comprises configuring the path such that, the displacement between, each of at least 80% of all positions along the path and at least one position at a distance along the path, wherein the distance along the path is more than the maximum distance, is less than or equal to the maximum distance.

This approach increases the likelihood that moving the ultrasound probe along the path will capture sufficient ultrasound data of the desired sub-surface region to wholly image the sub-surface region, e.g., to a sufficient level of detail to facilitate accurate reconstruction of the sub-surface region in the ultrasound image space.

The surface region may be a portion of an abdominal surface or pelvic surface of an individual. As an example, the sub-surface region may be a womb.

The identified path may take the shape of a grid. This provides an easy to follow and repeatable path for the ultrasound probe for an unskilled or inexperienced operator of the ultrasound probe to follow.

There is also proposed a computer program product comprising computer program code which, when executed on a computing device having a processing system, cause the processing system to perform all of the steps of any herein disclosed method.

There is also proposed a user interface system for providing ultrasound imaging guidance, the user interface system comprising a user interface and a processing system communicatively coupled to the user interface. The processing system is configured to carry out the functions achieved by performing any herein disclosed method. The user interface or the processing system may include or be provided with an image capturing device (e.g. a camera).

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a use-case scenario for proposed embodiments;
Fig. 2 is a flowchart illustrating a proposed method;
Fig. 3 is a flowchart illustrating a technique for determining a path for an ultrasound probe;
Fig. 4 is a flowchart illustrating a proposed method;
Fig. 5 is a flowchart illustrating a proposed method;
Fig. 6 is a flowchart illustrating a proposed method; and

Fig. 7 illustrates a user interface system.
Fig. 8 illustrates a schematic of a path in accordance with an embodiment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for identifying a recommended path for an ultrasound probe during an ultrasound imaging process. An image of a surface region, covering a desired sub-surface region, is received. The image is processed to identify the recommended path for the ultrasound probe that would capture ultrasound image data of the sub-surface region.

Embodiments are based on the realization that it is possible to predict a path over which an ultrasound probe should travel in order to capture ultrasound data of a particular region of interest.

Proposed approaches can be used in any environment in which ultrasound imaging of a medical subject takes place, but find particular advantage in environments with low resources and/or levels of expertise in operating an ultrasound probe.

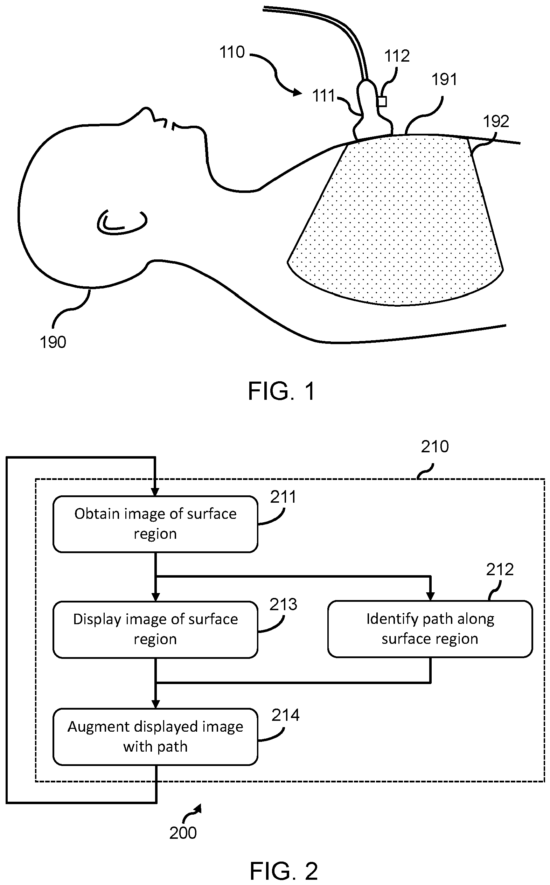

Fig. 1 schematically illustrates a scenario in which proposed embodiments can be used, for improved contextual understanding. In particular, Fig. 1 illustrates a subject 190 to undergo, undergoing or having undergone ultrasound imaging.

As it is well known, to perform ultrasound imaging, an ultrasound probe 110 is moved by an operator over a surface region 191 (e.g., the skin covering one or more internal organs) of the subject 190. The ultrasound probe iteratively generates ultrasound data, e.g., responsive to echoes of ultrasound waves emitted by the ultrasound probe. For instance, an ultrasound transducer 111 may emit ultrasound waves and produce ultrasound data responsive to any echoes that respond to the emitted ultrasound waves, following known procedures. The ultrasound data may comprise ultrasound image data (e.g., one or more 2D or 3D ultrasound images), or any suitable data from which a sequence of one or more 2D or 3D ultrasound images can be derived. For instance, raw ultrasound data may be provided by the ultrasound probe to an external ultrasound processing system (not shown) that processes the raw ultrasound data to produce ultrasound image data. The ultrasound (image) data represents a sub-surface region 192 of the individual, e.g., contains information about a part of the individual covered by the surface region 191.

By way of a working example only, the surface region may be a portion of an abdominal/pelvic surface of an individual. The sub-surface region may be or contain a womb or uterus, e.g., for performing a fetal ultrasound imaging procedure. Other suitable examples of surface regions and corresponding sub-surface regions will be apparent to the skilled person, and will depend upon the precise use-case scenario. For instance, in one example use-case scenario, the surface region is a surface of the chest, and the sub-surface region is or contains a thorax or chest cavity (e.g., at least the heart and/or lungs). As another example, the surface region is a surface of the pelvis, and the sub-surface region is or contains a reproductive organ (such as the uterus or womb). As yet another example, the surface region is a surface of the back, and the sub-surface region contains the soft tissue of the head and/or neck, e.g., at least the lymph glands.

It has been recognized that, in order to capture ultrasound (image) data, e.g., a sequence of (one or more) ultrasound images, of a desired sub-surface region 192, it is necessary to accurately move the ultrasound probe over the surface region 191 such that the ultrasound probe is appropriately positioned to capture all parts of the desired sub-surface region.

The present disclosure provides a mechanism for determining a path to be taken by an ultrasound probe 110 (along the surface region) in order to capture a particular desired sub-surface region. This determined path is then used to augment an image and/or video of the surface region.

This augmented image and/or video may be provided in advance of the ultrasound imaging procedure, e.g., to provide a recommended path for performing the ultrasound imaging procedure. In another approach, the augmented image and/or video may be provided during the course of the ultrasound imaging procedure, e.g., to provide active feedback for an operator of the ultrasound probe. In yet another approach, the augmented image and/or video may be provided after completion of the ultrasound imaging procedure, e.g., as a teaching aid to help guide the operator on potential improvements for moving of the ultrasound probe.

Fig. 1 also illustrates how the ultrasound probe 110 may comprise a movement sensor 112 for tracking or monitoring a movement of the ultrasound probe. Such a movement sensor may, for instance, comprise an accelerometer or gyroscope. Other example movements sensors are known in the art.

In the context of the present disclosure, a path is a route or trajectory, or combination of routes or trajectories, taken by the ultrasound probe when in contact with the surface region. A path may therefore be continuous (e.g., serpentine) or discontinuous/non-continuous (e.g., a grid).

Fig. 2 is a flowchart illustrating a computer-implemented method 200 for providing ultrasound imaging guidance. The computer-implemented method comprises performing an image display process 210, which may be iteratively performed.

The image display process 210 comprises a step 211 of receiving or obtaining an image of a surface region along which an ultrasound probe is to be moved during an ultrasound imaging procedure. The image is originally produced by a camera or other image capturing device positioned externally to the subject, and may be received from the image capturing device and/or a memory. The image of the surface region is therefore an image of the exterior of the subject on which ultrasound imaging is (e.g., to be or previously) performed.

The image display process 210 also comprises a step 212 of processing the image of the surface region to identify a path along the surface region for the ultrasound probe. The identified path is configured wherein, if the ultrasound probe is moved along said identified path during the ultrasound imaging procedure, the ultrasound probe would capture ultrasound data covering a desired sub-surface region. This identified path may alternatively labelled a "recommended path", which term may be used interchangeably throughout the remaining description.

In some examples, the identified path takes the shape of a grid. This provides a path that is easy for an inexperienced operator to follow or replicate. However, this shape is not essential, and the identified path may take other shapes (e.g., a serpentine shape or a set of parallel lines).

The image display process 210 also comprises a step 213 of controlling a user interface to display the image. Approaches for controlling a user interface to display an image are well known in the art, and are not described in detail for the sake of conciseness.

The image display process 210 also comprises a step 214 of augmenting the display of the image with a representation of the identified path that overlays the representation of the surface region in the displayed image. Approaches for augmenting an image with additional information, e.g., annotating the image, are also well known. As the image is processed in order to identify the path, the spatial relationship between parts of the image and the identified path are known. This facilitates trivial augmentation of the image with the identified path.

As previously mentioned, the image display process may be iteratively repeated.

In preferred examples, for each iteration of the image display process, the image received in each iteration comprises a different image in a sequence of images of the surface region. The sequence of images may, for instance, define a video of the surface region, and the different image may be the next image in the sequence of images.

In preferred examples, the image received by any iteration of the image display process is the most recently available image of a video stream of the surface region. This effectively facilitates provision of a dynamically updating video stream that is augmented to provide a representation of the determined path. This approach is particularly advantageous if the method is performed during an ultrasound imaging process, e.g., to provide immediate and ongoing feedback to an operator of the ultrasound probe.

However, in some examples, the method (or at least steps 213 and/or 214) can also/otherwise be performed before and/or after the ultrasound imaging process. Performing it before the ultrasound imaging process facilitates advance guidance of the ultrasound imaging process. Performing the method after the ultrasound imaging process facilitates the provision of feedback to the operator.

Thus, in some examples, at least step 214 (and optionally 213) are performed (only) before an ultrasound imaging process (representing an "initial mode"). In other examples, at least step 214 (and optionally 213) are performed (only) during an ultrasound imaging process (representing a "live mode"). In yet other examples, at least step 214 (and optionally 213) are performed (only) after the ultrasound imaging process (representing a "teaching mode").

In approaches in which the image display process is iteratively repeated, step 212 may be adapted to comprise adapting or updating the path based on the previously generated path for the previous iteration of the image display process. This may comprise, for instance, identifying a spatial shift between the image received in the current iteration of the image display process and the image received in a previous iteration of the image display process. The path may then undergo a corresponding spatial shift. Approaches for determining a spatial shift are well known in the art of image processing.

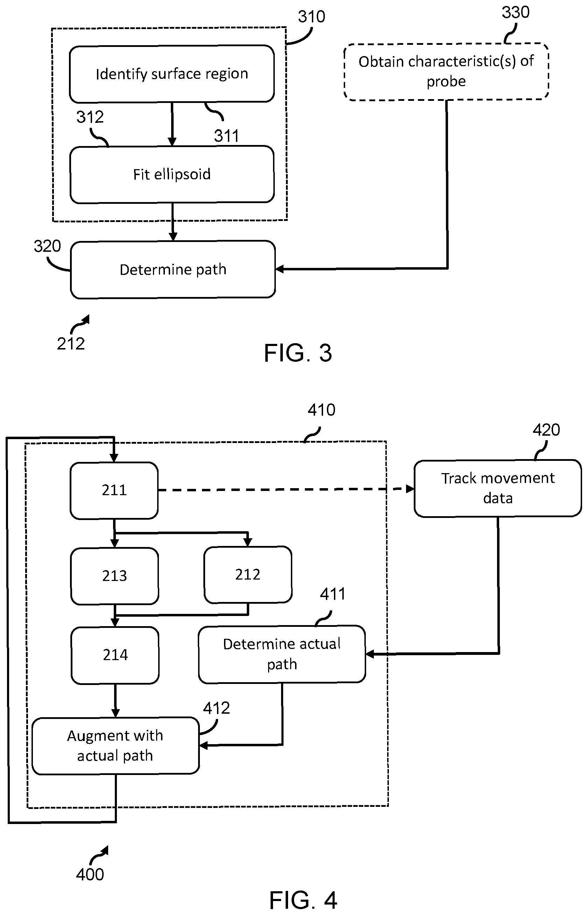

Fig. 3 is a flowchart illustrating an approach for performing step 212 for identifying a path along the surface region.

The proposed step 212 comprises a sub-step 310 of processing the image using an ellipsoid fitting algorithm to fit an ellipsoid over the portion of the image representing the surface region.

Sub-step 310 may be performed, for instance, by first identifying 311 the portion of the image that represents the surface region. This can be performed, for instance, using any suitably trained segmentation algorithm, such as a machine-learning algorithm and/or a deformable contour algorithm.

A wide variety of segmentation algorithms are known in the art, such as those mentioned by: Minaee, Shervin, et al. "Image segmentation using deep learning: A survey." IEEE transactions on pattern analysis and machine intelligence 44.7 (2021): 3523-3542; He, Lei, et al. "A comparative study of deformable contour methods on medical image segmentation." Image and vision computing 26.2 (2008): 141-163; or Ghosh, Swamendu, et al. "Understanding deep learning techniques for image segmentation." ACM Computing Surveys (CSUR) 52.4 (2019): 1-35. Any one or more of the identified segmentation techniques could be used and/or adapted for use for performing step 311, and others would be readily apparent to the suitable skilled person in the art.

Sub-step 310 may then perform an ellipsoid fitting step 312 that uses an ellipsoid fitting algorithm to fit an ellipsoid on the identified portion. Of course, step 312 may be omitted if, for instance, a segmentation algorithm is configured to directly produce an ellipsoid that identifies the portion of the image representing the surface region.

Step 312 may, for instance, be performed using the OpenCV fitEllipse function to fit an ellipse to the identified shape and position of the portion. Another approach makes use of a direct least square fitting ellipse method, using the edge of the identified portion of the image as the data points to which an ellipse is to be fitted. A wide variety of other ellipsoid fitting techniques will be readily apparent to the skilled person.

In another example, sub-step 310 may be performed using a geodesic fitting technique that makes use of one or more single image depth estimation techniques that have been developed. One example of a suitable technique is put forward by Mertan, Alican, Damien Jade Duff, and Gozde Unal. "Single image depth estimation: An overview." Digital Signal Processing (2022): 103441.

By way of working example, it is known for an ellipsoid equation to be defined in the form:

$$\frac{(x-g)^2}{a^2} + \frac{(y-h)^2}{b^2} + \frac{(z-i)^2}{c^2} = 1 \qquad (1)$$

Every point in the ellipsoid should satisfy this equation or (from an estimation paradigm) the absolute error of the actual points from this equation should be minimized with respect to the best estimates of the parameters. Depth estimates can be used to estimate the parameters of this equation assuming that the z-origin corresponds to roughly the foremost region of the image.

The proposed step 212 further comprises a sub-step 320 of identifying the path based at least on a size of the fitted ellipsoid. For instance, based on an area of the detected surface region (e.g., as represented by the fitted ellipsoid), the number and/or position of one or more sweep lines for a path to be taken by an ultrasound probe can be identified. For instance, there may be a predetermined mapping between size of the fitted ellipsoid and number of (recommended) sweep lines.

In some examples step 320 comprise using depth information, together with the fitted ellipsoid, to identify the size of the surface region. In particular, depth information may be used to define distances between different points within the image of the surface area. This facilitates identification of the size of a surface region (e.g., the size of the fitted ellipsoid) in real terms. The identified size of the surface region may be used to define or determine the path to be taken by the ultrasound probe to capture ultrasound data of the desired sub-region. Depth information may, for instance, be estimated using any suitable depth information technique, such as that proposed by Mertan, Alican, Damien Jade Duff, and Gozde Unal. "Single image depth estimation: An overview." Digital Signal Processing (2022): 103441.

There may be a predetermined mapping between size (whether relative size in an image or determined corresponding real size) of the fitted ellipsoid and number of (recommended) sweep lines and/or pattern of the recommended sweep lines. A sweep line is a movement of the ultrasound probe along a single curve or line, as is known in the art.

In examples, information about the ultrasound probe (and/or ultrasound system that uses the ultrasound probe) is used to calculate or determine the path. Thus, step 320 may comprise using one or more known characteristics of the ultrasound probe to determine the path. Accordingly, the step 212 may comprise a sub-step 330 of obtaining one or more (known) characteristics of the ultrasound probe.

As a working example, the one or more (known) characteristics of the ultrasound probe comprises a recommended or maximum distance. A recommended/maximum distance may, for instance, define a maximum recommended distance (e.g., as recommended by the manufacturer) between two sweep lines of an ultrasound probe. The maximum distance may, for instance, define a maximum allowable distance between two positions of the ultrasound probe that each image a sub-surface region whose sides wholly overlap. In this way, the maximum distance may define a maximum allowable distance between a first position of the ultrasound probe, for imaging a first sub-surface region, and a second position of the ultrasound probe, for imaging a second sub-surface region, in which one side of the first sub-surface region wholly overlaps the second sub-surface region. For example, the right edge of an ultrasound image covering a first sub-surface region may wholly overlap with the left edge of a second ultrasound image covering a second sub-surface region so as to avoid any imaging gap between the first and second images. In other examples, a side may be a side of an image, for example a left half, right half, top half or bottom half, and the right side of a first image is to overlap with the left side of a second image. This overlap may be wholly or partially.

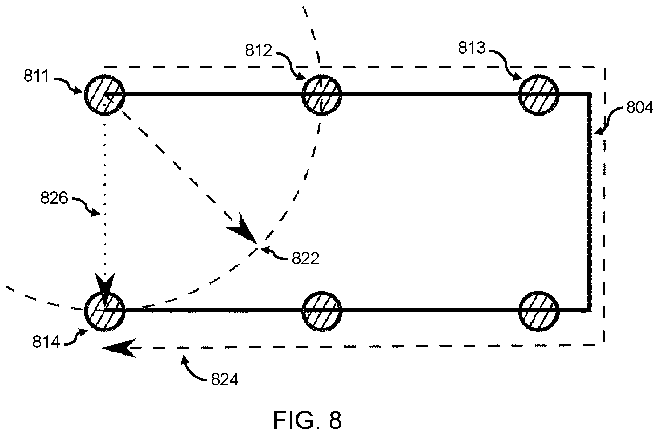

Fig. 8 illustrates the position 811 that a probe would for example take for imaging a first subsurface region and a position 812 that a probe would take for imaging a second sub-surface region.

By choosing a path using a recommended or maximum distance facilitates identification of a path that captures sufficient ultrasound data to image the majority of the entirety of the desired sub-surface region.

For instance, the step 320 of identifying the path may comprises configuring the path according to Fig 8, such that, between each of at least 80% of all positions 811, 812, 813, 814 along the path 804 and at least one position 814 at a distance 824 along the path 804, wherein the distance 824 along the path is more than the maximum distance 822, the displacement (i.e., shortest distance between two points) is less than or equal to the maximum distance. In other words, with reference to exemplary Fig. 8, during an imaging procedure, a probe may follow a path 804 through imaging positions 811, 812, 813, ... until reaching imaging position 814. The distance travelled by the probe between each of these imaging positions, 811 to 812, 812 to 813, ..., may be less than the maximum distance 822. The total distance travelled by the probe to get from position 811 to 814 is thus the distance 824 along the path 804, which is a distance that is greater than the maximum distance 822. The displacement of the probe at position 814 with respect to 811, is however less than or equal to the maximum distance 822. This effectively facilitates the identification of a path that will image no less than 80% of the desired sub-surface region.

Fig. 4 is a flowchart illustrating a computer-implemented method 400 providing further optional steps. The method 400 comprises performing an image display process 410 that comprises the previously described steps 211, 212, 213, 214.

The computer-implemented method 400 further comprises a step 420 of tracking a movement of the ultrasound probe during the ultrasound imaging procedure. Approaches for tracking a movement of an ultrasound probe are known, and may, for instance, comprise using movement data captured by a movement sensor in or on the ultrasound probe to track the movement of the ultrasound probe.

In a preferred example, step 420 comprises tracking the movement of the ultrasound probe with respect to the image received in step 211. This can be achieved, for instance, by processing the image received in step 211 to identify the relative location of the image representation of the ultrasound probe within the image. In this way, the tracked (real) path can be directly produced in a same frame of reference as the recommended path.

Put another way, any images of the surface region obtained during the ultrasound imaging procedure will contain a representation of the ultrasound probe. Thus, it is possible to track the location of the ultrasound probe using an image produced by the same camera that is/was used to determine the recommended path. Approaches for tracking an ultrasound probe through a sequence of images may make use of one or more optical flow algorithms (as the probe will move substantially more than other aspects of the image) and/or object recognition algorithms (e.g., segmentation algorithms designed to identify the location of the ultrasound probe).

In another example, the movement of the ultrasound probe may be tracked in a separate co-ordinate system/-space (i.e., a difference frame of reference). Technology for tracking of an object in three-dimensional space is known and includes, for example, accelerometer-based tracking, electromagnetic tracking, infrared (light) tracking, and mechanical positioning systems. Approach for tracking a movement of (and therefore facilitating the tracking of a path taken by) an ultrasound probe are disclosed by the US Patent Applications having Publication numbers US 2021/186622 A1; US 2013/245428 A1 and US 2010/268072 A1.

The image display process 410 may further comprise a step 411 of processing the tracked movement of the ultrasound probe to identify an actual path along the surface region taken by the ultrasound probe. This can be performed, for instance, be iteratively recording the movement to track the path or route taken by the ultrasound probe along the surface.

The identified actual path may be defined with respect to the image and/or three-dimensional space.

The image display process 410 may further comprise a step 412 of augmenting the display of the image with a representation of the identified actual path that overlays the representation of the surface region in the display image.

If the actual path is defined in a three-dimensional space (e.g., and not directly with respect to the display image), then the spatial relationship between the display image and the actual path can be readily determined using standard procedures.

For instance, the co-ordinate system for the tracked (real) path can be mapped or spatially registered to the frame of reference of the display image. This facilitates spatial registration of the real path to the display image.

In an example, the (display) image may be spatially registered to three-dimensional space (for tracking the real path of the probe) using a calibration process. For instance, the probe may be positioned at a plurality of predetermined positions on the surface region. For each of these predetermined positions, the position of the probe in the three-dimensional space is recorded. Similarly, for each of these predetermined positions, the location that represents the predetermined position (e.g., as identified by a segmentation process) within the image is recorded. For each predetermined position, the location in the three-dimensional space is correlated with the location in the image. The image is then mapped or warped to the three-dimensional space. This mapping may make use of distance information, e.g., derived from predicted depth information for the image. In this way, the relative spatial registration between a position in the three-dimensional space and a corresponding position represented in the image can be determined.

An example makes use of an assumption that the invention is employed to process a sequence of images (e.g., a video stream) of the subject's area of interest with negligible camera motion. The sequence would start with a frame that doesn't have a representation of a probe or (even if there is a probe) the probe would be the moving part in an otherwise static (except for apparent movement caused due to small camera movements) background. A motion estimation method, such as optical flow, will be able to localize the probe pretty accurately. Known image registration methods work reasonably well to determine the shift in coordinates if there is a negligible change to the camera position in fixing the background portion. Thus, it is possible estimate or roughly get the net displacement (e.g., displacement of background in frame of interest from the reference we get via image registration and displacement of probe with respect to the previous frame via optical flow methods) of the probe.

In an example, an image-based detector can be used. The image-based detector may detect the probe in the image along with other key markers or predetermined locations of the surface region (e.g., bellybutton and/or other markers on the abdomen etc.) to get a relative position of the probe with respect to each marker. This can be used to relax the small camera movement constraint while these marker coordinates are mapped to special values like the origin etc.

The preceding two methods would make the measurements somewhat independent of the current frame.

Augmenting the image in this way allows for identification and/or comparison between a recommended path for the ultrasound probe and an actual path. This is useful, for instance, for providing direct feedback during an ultrasound imaging process or for providing post-procedure feedback, e.g., for automated feedback to an operator of the ultrasound probe on their performance.

In some examples, at least step 412 (and optionally step 214 and optionally step 213) is performed (only) before an ultrasound imaging process (representing an "initial mode"). In other examples, at least step 412 (and optionally step 214 and optionally step 213) is performed (only) during an ultrasound imaging process (representing a "live mode"). In yet other examples, at least at least step 412 (and optionally step 214 and optionally step 213) is performed after the ultrasound imaging process (representing a "teaching mode").

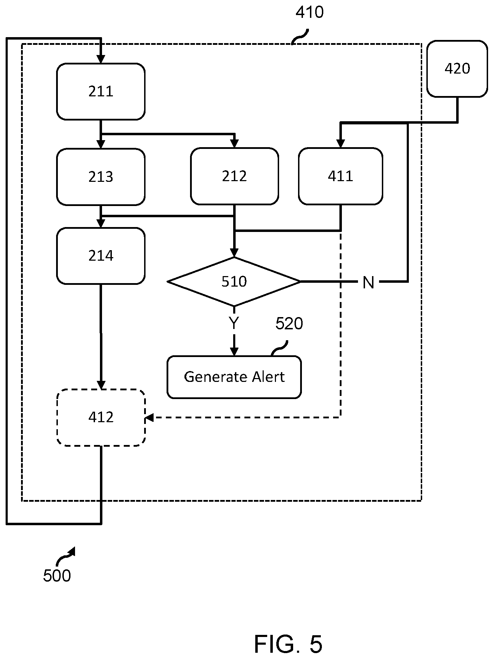

Fig. 5 is a flowchart illustrating a variant to the computer-implemented method 500 described with reference to Fig. 4. Steps that refer to the same features are identified with the same reference numerals. The method 500 is designed to be performed during the ultrasound imaging process, as it provides direct feedback to the operator of the ultrasound probe.

In this approach, a determination step 510 is performed to determine whether or not the identified (recommended) path differs from the actual path by more than a predetermined amount, e.g., whether an average distance between the actual path and the identified (recommended) path is greater than some predetermined value or whether a distance between any point on the actual path and the identified (recommended) path is greater than the predetermined value.

Step 510 may require the spatial registration of the actual path to the recommended path (or vice versa) to facilitate the determination of a distance between the two paths. Spatial registration may be required, for instance, if the two paths are not originally defined in a same co-ordinate system, e.g., with respect to the image used to derive the recommended path.

As the recommended path may be defined with respect to the image, and the real path may be defined with respect to a particular three-dimensional space, e.g., if tracked in the three-dimensional space, it is possible to register the real and recommended paths together based on previously described approaches for registering the image to three-dimensional space.

Response to a positive determination in step 510, a user-perceptible alert may be generated in a step 520. A user-perceptible alert comprises any suitable sensory output (e.g., a visual, audio and/or haptic output) that can be perceived by an operator (e.g., of the ultrasound probe). Examples include an audible alert (e.g., a buzzing), a visible alert (e.g., a flashing light or a particular color) or a haptic alert (e.g., a vibration - which may be provided at the ultrasound probe). Otherwise, the method may revert back to step 411.

This approach allows for direct feedback on the accuracy or suitability of the path taken by the ultrasound probe during the ultrasound imaging process.

In some embodiments, step 412 can be omitted. Thus, step 412 is optional in some embodiments of the computer-implemented method 500.

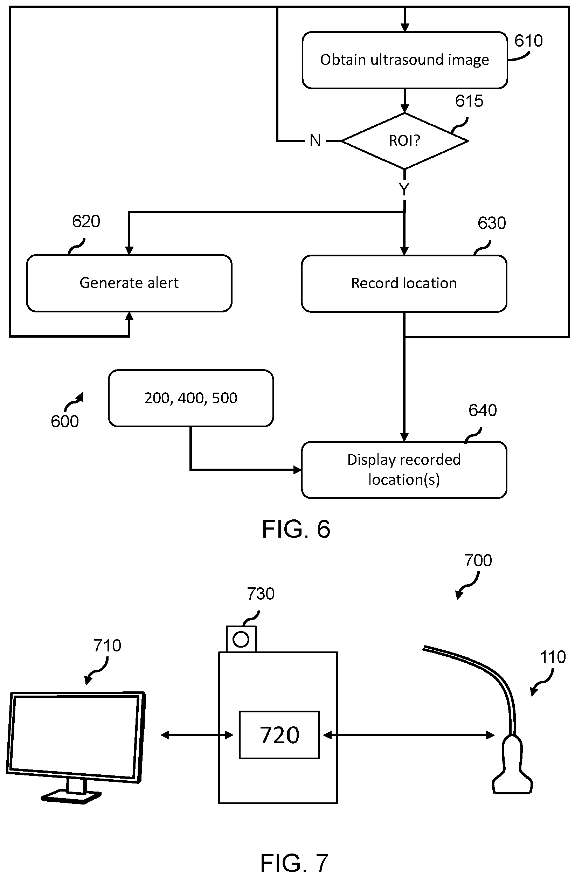

Fig. 6 is a flowchart that illustrates some further optional features. More particularly, Fig. 6 illustrates a computer-implemented method 600 that comprises any previously described computer-implemented method 200, 400, 500.

The computer-implemented method further 600 further comprises a step 610 of iteratively receiving an ultrasound image from the ultrasound probe during the ultrasound imaging procedure.

The computer-implemented method 600 further comprises, for each ultrasound image: processing 615 the ultrasound image to determine whether or not a region of interest (ROI) is captured in the ultrasound image.

Step 615 may be performed, for instance, using one or more image classification algorithms. One examples of such an approach is disclosed by the European Patent Applications having publication number EP 3,639,751 A1. Other examples will be readily apparent to the skilled person, and may employ any suitable classification algorithm for classifying an image, e.g., configured to determine the presence or absence of one or more anatomical/structural elements (e.g., anatomical organs) within an image. Example classification algorithms may make use of one or more machine-learning algorithms or the like.

In some examples, the method 600 comprises, responsive to determining (in step 615) that a region of interest is captured, controlling 620 the user interface to provide a user-perceptible alert that the ultrasound probe is capturing an ultrasound image of a region of interest. Approaches for controlling a user interface to perform step 620 are well known in the art. A number of example approaches have been previously described with reference to a similar step 520.

In some examples, the method 600 comprises, responsive to determining (in step 615) that a region of interest is captured, recording 630 a location at which the region of interest was captured.

If the movement of the ultrasound probe is tracked, this may comprise defining or determining the location of the ultrasound probe within the co-ordinate system used to track the ultrasound probe.

If images of the surface region are iteratively captured throughout the ultrasound imaging procedure, then step 630 may comprise recording or saving the image captured closest to the time at which the region of interest was captured in ultrasound image data. As the ultrasound probe is positioned on the surface region, an image of the surface region will contain a representation of the ultrasound probe with respect to the surface region. This allows the relative location of the ultrasound probe to be identified.

In some examples, the method 600 further comprise, e.g., after completion of the scan, augmenting the display of the surface region with a display of the identified location(s). This can, for instance, be performed by mapping a recorded location to a location on the displayed image, e.g., using a previously described technique.

Steps 630 and 640 facilitate identification of the location at which interesting ultrasound images were obtained. This can provide valuable information to a clinician. For instance, such locations may identify regions that may benefit from further ultrasound imaging, e.g., to obtain more information. As another example, information on the locations can identify anatomical characteristics, e.g., the location of certain anatomical structures or the orientation of anatomical structures such as a fetus. By way of example, if the regions of interest include a fetal head and a fetal heart/bottom, then displaying the locations at which ultrasound images of these regions of interest were captured will facilitate identification of fetal orientation.

Fig. 7 illustrates a user interface system 700 for providing ultrasound imaging guidance. The user interface system comprises a user interface 710 and a processing system 720, communicatively coupled to the user interface.

The processing system 720 is configured to perform an image display process according to any of the methods described herein. In particular, a method comprising the steps of receiving or obtaining an image of a surface region along which an ultrasound probe is to be moved during an ultrasound imaging procedure; processing the image of the surface region to identify a path along the surface region for the ultrasound probe, wherein if moved along the identified path during the ultrasound imaging procedure, the ultrasound probe would capture a sequence of ultrasound images covering a desired sub-surface region; controlling the user interface to display the image; and augmenting the display of the image with a representation of the identified path that overlays the representation of the surface region in the displayed image.

The processing system may receive the image(s) of the surface region from a camera 730 that captures an image (e.g., a visible light image) of the surface region. The camera may be integrated into a same device or product that carries the processing system (e.g., a mobile phone, tablet, laptop and so on). Alternatively, the camera may be a separate component, e.g., an additional or separate camera. The camera may alternatively be integrated in the user interface 710. The camera 730 may form part of the user interface system 700.

The processing system 720 may be further configured to communicate with an ultrasound probe 110, e.g., to receive ultrasound image(s) produced by the ultrasound probe or generate ultrasound images using ultrasound data produce by the ultrasound probe. Thus, the processing system 720 may form part of an ultrasound imaging system. The ultrasound probe 110 may form part of the user interface system 700.

Although illustrated as a separate element, in some examples the user interface 710 forms part of the same device or product that carries the processing system, e.g., a user interface (e.g., screen and optionally speaker) of a smartphone, tablet or laptop.

The skilled person would be readily capable of developing the processing system for carrying out any herein described method. Thus, each step of the flow chart may represent a different action performed by the processing system, and may be performed by a respective module of the processing system.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code (i.e. instructions) for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. Measures recited in mutually different dependent claims may advantageously be combined.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (200, 400, 500, 600) for providing ultrasound imaging guidance, the computer-implemented method comprising performing an image display process (210, 410) comprising:

   receiving (211) an image of a surface region (191) along which an ultrasound probe (110) is to be moved during an ultrasound imaging procedure;
   processing the image of the surface region to identify (212) a path (804) along the surface region for the ultrasound probe, wherein if moved along the identified path during the ultrasound imaging procedure, the ultrasound probe would capture a sequence of ultrasound images covering a desired sub-surface region (192);
   controlling a user interface to display (213) the image; and
   augmenting (214) the display of the image with a representation of the identified path that overlays the representation of the surface region in the displayed image.

2. The computer-implemented method of claim 1, comprising iteratively performing the image display process, wherein the image received in each iteration of the image display process comprises a different image in a sequence of images of the surface region.

3. The computer-implemented method of claim 2, wherein the next image in a sequence of images comprises the most recently available image of a video stream of the surface region.

4. The computer-implemented method of any of claims 1 to 3, further comprising tracking (420) a movement of the ultrasound probe during the ultrasound imaging procedure, wherein the image display process (210, 410) further comprises:

processing the tracked movement of the ultrasound probe to identify (411) an actual path along the surface region taken by the ultrasound probe; and

augmenting (412) the display of the image with a representation of the identified actual path that overlays the representation of the surface region in the display image.

5. The computer-implemented method of claim 4, wherein the step of processing the tracked movement of the ultrasound probe comprises using movement data captured by a movement sensor (112) in the ultrasound probe to track the movement of the ultrasound probe.

6. The computer-implemented method of any of claims 1 to 5, further comprising:

iteratively receiving (610) an ultrasound image captured by the ultrasound probe during the ultrasound imaging procedure; and
for each ultrasound image:

processing (615) the ultrasound image to determine whether or not a region of interest is captured in the ultrasound image; and
responsive to determining that a region of interest is captured, controlling the user interface to provide (620) a user-perceptible alert that the ultrasound probe is capturing an ultrasound image of a region of interest.

7. The computer-implemented method of any of claims 1 to 6, wherein the step of processing the image of the surface region comprises:

processing (310) the image using an ellipsoid fitting algorithm to fit an ellipsoid over the portion of the image representing the surface region; and
identifying (320) the path based at least on a size of the fitted ellipsoid.

8. The computer-implemented method of claim 7, wherein the step of identifying the path based on at least a size of the fitted ellipsoid comprises using one or more known characteristics of the ultrasound probe.

9. The computer-implemented method of claim 8, wherein the one or more known characteristics of the ultrasound probe comprises a maximum distance (822), the maximum distance being a maximum allowable distance between a first position (811) of the ultrasound probe, for imaging a first sub-surface region, and a second position (812) of the ultrasound probe, for imaging a second sub-surface region, in which one side of the first sub-surface region wholly overlaps the second sub-surface region.

10. The computer-implemented method of claim 9, wherein the step of identifying the path comprises configuring the path such that, the displacement (826) between

- each of at least 80% of all positions (811, 812, 813, 814) along the path (804) and
- at least one position (814) at a distance (824) along the path (804),
wherein the distance along the path is more than the maximum distance (822),

is less than or equal to the maximum distance (822).

11. The computer-implemented method of any of claims 1 to 10, wherein the surface region is a portion of an abdominal surface or pelvic surface of an individual.

12. The computer-implemented method of any of claims 1 to 11, wherein the sub-surface region is a womb.

13. The computer-implemented method of any of claims 1 to 12, wherein the identified path takes the shape of a grid.

14. A computer program product comprising computer program code which, when executed on a computing device having a processing system (720), cause the processing system to perform the method according to any of claims 1 to 13.

15. A user interface system (700) for providing ultrasound imaging guidance, the user interface system comprising a user interface (710) and a processing system (720) communicatively coupled to the user interface, the processing system being configured to perform the method according to any of claims 1 to 13.

110
111
112
191
192
190

FIG. 1

210

Obtain image of surface region
211

Display image of surface region
213

Identify path along surface region
212

Augment displayed image with path
214

200

FIG. 2

FIG. 3

FIG. 4

FIG. 5

FIG. 6

FIG. 7

FIG. 8

## EUROPEAN SEARCH REPORT

Europäisches
Patentamt
European
Patent Office
Office européen
des brevets

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/024030 A1 (LACHAINE MARTIN [CA] ET AL) 22 January 2009 (2009-01-22) | 1,4,5,14 | INV. A61B8/08 |
| A | * paragraphs [0001], [0008] – [0012], [0019] – [0029]; claims; figures * | 2,3, 6-13,15 | A61B8/00 |
| A | US 2014/114193 A1 (ANTHONY BRIAN W [US] ET AL) 24 April 2014 (2014-04-24) * paragraph [0046]; claims; figures * | 1-15 | |
| A | US 2020/375546 A1 (SHOUDY DAVID ANDREW [US] ET AL) 3 December 2020 (2020-12-03) * paragraph [0038]; claims; figures * | 1-15 | |
| A | US 2020/367860 A1 (ROUET LAURENCE [FR] ET AL) 26 November 2020 (2020-11-26) * paragraphs [0132] – [0147]; claims; figures * | 1-15 | |
| A | WO 2013/055611 A1 (TRACTUS CORP [US]) 18 April 2013 (2013-04-18) * paragraphs [0071] – [0072] * | 1-15 | |

TECHNICAL FIELDS
SEARCHED      (IPC)

A61B

The present search report has been drawn up for all claims

| Place of search | Date of completion of the search | Examiner |
|---|---|---|
| Munich | 21 November 2023 | Mundakapadam, S |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

......................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 18 0865

This annex lists the patent family members relating to the patent documents cited in  the above-mentioned European search report.
The members are as contained in the European Patent Office EDP file on
The European Patent Office is in no way liable for these particulars which are merely given for the purpose of information.

21-11-2023

| Patent document cited in search report | | | Publication date | Patent family member(s) | | | Publication date |
|---|---|---|---|---|---|---|---|
| US 2009024030 | A1 | | 22-01-2009 | US | 2009024030 | A1 | 22-01-2009 |
| | | | | WO | 2009012576 | A1 | 29-01-2009 |
| US 2014114193 | A1 | | 24-04-2014 | NONE | | | |
| US 2020375546 | A1 | | 03-12-2020 | CN | 112022201 | A | 04-12-2020 |
| | | | | US | 2020375546 | A1 | 03-12-2020 |
| US 2020367860 | A1 | | 26-11-2020 | CN | 111629668 | A | 04-09-2020 |
| | | | | EP | 3485816 | A1 | 22-05-2019 |
| | | | | EP | 3713494 | A1 | 30-09-2020 |
| | | | | JP | 2021503992 | A | 15-02-2021 |
| | | | | US | 2020367860 | A1 | 26-11-2020 |
| | | | | WO | 2019101609 | A1 | 31-05-2019 |
| WO 2013055611 | A1 | | 18-04-2013 | AU | 2012321147 | A1 | 17-04-2014 |
| | | | | CA | 2851590 | A1 | 18-04-2013 |
| | | | | CN | 104168837 | A | 26-11-2014 |
| | | | | EP | 2765918 | A1 | 20-08-2014 |
| | | | | JP | 2014528347 | A | 27-10-2014 |
| | | | | KR | 20140128940 | A | 06-11-2014 |
| | | | | US | 2013225986 | A1 | 29-08-2013 |
| | | | | US | 2018132722 | A1 | 17-05-2018 |
| | | | | WO | 2013055611 | A1 | 18-04-2013 |

EPO FORM P0459

For more details about this annex : see Official Journal of the European Patent Office, No. 12/82

**EP 4 480 424 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021186622 A1
- US 2013245428 A1
- US 2010268072 A1
- EP 3639751 A1

**Non-patent literature cited in the description**

- **MINAEE, SHERVIN et al.** Image segmentation using deep learning: A survey. *IEEE transactions on pattern analysis and machine intelligence*, 2021, vol. 44.7, 3523-3542
- **HE, LEI et al.** A comparative study of deformable contour methods on medical image segmentation. *Image and vision computing*, 2008, vol. 26.2, 141-163
- **GHOSH, SWAMENDU et al.** Understanding deep learning techniques for image segmentation. *ACM Computing Surveys (CSUR)*, 2019, vol. 52.4, 1-35
- **MERTAN, ALICAN ; DAMIEN JADE DUFF ; GOZDE UNAL**. Single image depth estimation: An overview. *Digital Signal Processing*, 2022, 103441
- **MERTAN, ALICAN, DAMIEN JADE DUFF ; GOZDE UNAL**. Single image depth estimation: An overview. *Digital Signal Processing*, 2022, 103441